# EUROPEAN PATENT SPECIFICATION

(11) **EP 4 479 127 B1**
(45) Date of publication and mention of the grant of the patent: **28.01.2026**
(21) Application number: 23702344.5
(22) Date of filing: 31.01.2023
(51) Int. Cl.: A61N 1/05

(54) **PADDLE LEAD**
PADDELELEKTRODE
DÉRIVATION À PALETTE

(30) Priority: 17.02.2022 EP 22157183
(43) Date of publication of application: 25.12.2024
(73) Proprietor: BIOTRONIK SE & Co. KG, 12359 Berlin (DE)
(72) Inventor: RUMP, Jens, 12049 BERLIN (DE); ZICKERT, Christian, 79618 RHEINFELDEN (DE); BUCHNER, Dagmar, 10245 BERLIN (DE); ROMBERG, Jan, 12347 BERLIN (DE)
(74) Representative: Biotronik Corporate Services SE
(86) International application number: PCT/EP2023/052318
(87) International publication number: WO 2023/156191

(56) References cited:
- WO-A1-2013/075171
- WO-A2-99/55411
- US-A1- 2011 130 805
- US-A1- 2020 316 371

## Description

The present invention relates to a paddle lead according to claim 1 as well as to a method for manufacturing such a paddle lead according to claim 15.

Paddle leads are regularly used for spinal cord stimulation (SCS) and are placed for this purpose in the epidural space close to the spinal cord.

The term "paddle lead" originates from the flattened, paddle-like shape of these leads. Paddle leads carry an arrangement of multiple evenly or unevenly distributed electrode poles (also referred to as contact elements) on their surface for emission of electrical signals into and/or reception of electrical signals from surrounding tissue.

Paddle leads are often implanted in a surgical manner close to the height of the final site of action. For this purpose, parts of a vertebral body in this region are removed to provide necessary space for implantation.

However, it is also known to implant paddle leads in a minimally invasive way like percutaneous leads. To allow such minimally invasive implantation, it is necessary to compress the paddle lead to a delivery state in which the outer diameter of the paddle lead is reduced so that the paddle lead can be guided through an implantation catheter used for minimally invasive implantation. Once positioned at its intended site of implantation, the paddle lead is released from the implantation catheter and transferred from its (collapsed) delivery state into its (expanded) implantation state.

US 6,205,361 B1 describes such a collapsible paddle lead. Here, a shape memory element is placed in the center of a cylindrical edge portion of the paddle lead. The shape memory element has a shape that maintains the open, paddle shape of the paddle lead and thus biases the paddle lead to the expanded implantation state.

US 2010/0082086 A1 describes a paddle lead comprising an elastically deformable elongated plate that can be releasably rolled into a substantially cylindrical form for delivery purposes. After completing the delivery, the cylinder unrolls again so that a paddle to be implanted is present.

US 2012/0283808 A1 describes a paddle lead having a frame of rigid material and comprising a spring member adapted to bias the frame to assume an implantation shape. In addition, elastic material is disposed across an interior surface area defined by the frame. In this context, a plurality of different shapes for the elastic material is disclosed in this US patent application.

US 2014/0200639 A1 describes a self-expanding lead with a plurality of expandable arms that form - in their expanded state - a complex geometry. The arms include a resilient member that is biased to flex the arms from a collapsed state to an expanded state. These resilient members form an integral part of the arms to be expanded.

WO2013075171 A1 describes an electrode assembly for an active implantable medical device can be delivered by catheter but expands to become a paddle electrode once implanted. The electrode assembly comprises a support member carrying wires for electrically connecting a control unit to electrodes of the electrode assembly. At least one, and usually two resilient deformable paddle wings are mounted to the support member. The paddle wings can be furled close to the support member under a deformation force to permit implantation via an introducer. The paddle wings resiliently unfurl away from the support member upon release of the deformation force. The paddle wings bear rows and columns of electrodes, and the electrode assembly as a whole has sufficient longitudinal rigidity for implantation via an introducer.

It is an object of the present invention to provide a paddle lead for implantation in the epidural space through an insertion tool that can be easily collapsed for delivery purposes and expanded for implantation purposes and that can be easier and cheaper produced than paddle leads known from prior art.

This object is achieved with a paddle lead for implantation in the epidural space through an insertion tool having the claim elements of claim 1. Such a paddle lead comprises a lead body. The lead body, in turn, comprises a plurality of conductors. These conductors extend from a proximal portion of the lead body to a distal portion of the lead body. The paddle lead further comprises a paddle structure that is arranged distally of the lead body and is electrically coupled to the distal portion of the lead body. The paddle structure has a longitudinal extension direction. The paddle structure further comprises a first paddle wing and a second paddle wing. The first paddle wing and the second paddle wing are movable with respect to each other and can be present in an expanded state (implantation state) or in a collapsed state (delivery state).

A plurality of first electrode poles (contact elements) is arranged on the first paddle wing. In this context, each of the first electrode poles is electrically coupled to at least one (and typically exactly one) of the plurality of conductors. Likewise, a plurality of second electrode poles is arranged on the second paddle wing. Also in this case, each of the second electrode poles is electrically coupled to at least one (and typically exactly one) of the plurality of conductors.

The paddle structure further comprises at least one resilient element that is connected to the first paddle wing and/or the second paddle wing. In this context, the at least one resilient member biases the first paddle wing and/or the second paddle wing to the expanded state.

According to an aspect of the present invention, the at least one resilient element has a longitudinal extension direction running exclusively along the longitudinal extension direction of the paddle structure. In addition, the at least one resilient element has a wall that surrounds either alone or together with another part of the paddle structure a hollow space. In this context, the hollow space also extends exclusively along the longitudinal extension direction of the paddle structure.

Since the resilient element has a longitudinally extending shape, it can be particularly easy arranged between the first paddle wing and the second paddle wing. This facilitates manufacturing of the paddle lead. Furthermore, the hollow space surrounded by the resilient element (either alone or together with another part of the paddle structure) enables that the resilient element can be very favorably compressed and does not require much space in its compressed state. This facilitates collapsing of the first paddle wing and the second paddle wing in order to form a collapsed state of the paddle structure.

In contrast to prior art solutions in which individual elastic elements are placed side-by-side to each other, the use of the specifically designed resilient element according to an aspect of the present invention does not require complicated arrangements of different elements, but rather enables an easy insertion of the resilient element along the longitudinal extension direction of the paddle structure.

Since the resilient element already biases the first paddle wing and/or the second paddle wing to the expanded state, it is no longer necessary to provide additional spring members for transferring the paddle structure into its expanded state. Therefore, the paddle structure itself can be manufactured much cheaper than according to prior art solutions.

In an embodiment, the hollow space is accessible only by a first end of the at least one resilient element and/or a second end of the at least one resilient element, wherein the second end lies opposite the first end along the longitudinal extension direction of the at least one resilient element.

In an embodiment, the paddle structure and/or the paddle lead does not comprise a spring member other than the resilient element for biasing the first paddle wing and/or second paddle wing into its expanded state.

In an embodiment, the at least one resilient element is made from a different material than the first paddle wing and the second paddle wing. To give an example, the first paddle wing and the second paddle wing may comprise a memory shape material such as nitinol. **In** such a case, the at least one resilient element is not made from nitinol. To give another example, the first paddle wing and the second paddle wing may be made from a non-elastic, rigid plastic material. In such a case, the at least one resilient element is made from a different plastic material or from a resilient metallic material.

In an embodiment, the at least one resilient element is made from an elastic plastic material. Silicone and polyurethane are particularly appropriate elastic plastic materials for this purpose. Such an elastic plastic material enables a particularly easy and cheap manufacturing of the at least one resilient element. Furthermore, the overall weight of the paddle lead is reduced in case of using a plastic material for the resilient element. This enhances the user-friendliness of the paddle lead since the dead weight of the paddle lead is reduced compared to paddle leads making excessive use of metal for the individual components.

In an embodiment, the at least one resilient element has a circular or elliptical cross-section. In this embodiment, the at least one resilient element has the shape like a hose. Such a hose-like resilient element can be manufactured in a particularly simple way and can be introduced between the first paddle wing and the second paddle wing in a particularly simple manner during the manufacturing process of the paddle lead. In addition, due to the hollow space within the hose-like shape (surrounded by the wall of the resilient element), the resilient element can be compressed to a particularly small volume so that it does not impart a collapsing of the paddle lead to its collapsed delivery state.

In an embodiment, the at least one resilient element has a cross section of a segment of a circle or of a segment of an ellipse. In this embodiment, the at least one resilient element has the shape of a circumferential section of a hose. In this case, a hollow space is formed by the at least one resilient element together with another part (e.g., the first paddle wing and/or the second paddle wing) to surround a hollow space. Also in this case, a particularly favorable collapsing of the at least one resilient element is made possible. Furthermore, also such a circumferential section of a hose can be manufactured in a particularly simple and cheap way.

In an embodiment, the first paddle wing and the second paddle wing are connected with each other in the proximal portion of the first paddle wing and of the second paddle wing. At the same time, they are unconnected in a distal portion of the first paddle wing and of the second paddle wing. This results in a bifurcated structure of the first paddle wing and the second paddle wing, i.e., of the paddle structure, with freely movable distal ends of the first paddle wing and the second paddle wing. This facilitates the execution of movements during collapsing and expanding of the paddle structure.

In an embodiment, the at least one resilient element is mechanically linked with the first paddle wing and with the second paddle wing. In this embodiment, the at least one resilient element biases both the first paddle wing and the second paddle wing to their respective expanded state. In the simplest arrangements, only a single resilient element is necessary to achieve a transition of the first paddle wing and the second paddle wing from their collapsed state into their expanded state by acting upon them with an appropriate force. In this and in all other embodiments, the mechanical linkage can be realized, e.g., by gluing the resilient element to the first paddle wing and/or the second paddle wing.

In an embodiment, the paddle structure additionally comprises a ligament extending along the longitudinal extension direction of the paddle structure. In this context, the ligament is arranged between the first paddle wing and the second paddle wing. Furthermore, the at least one resilient element is also connected to the ligament. Such a ligament enables a Y-shaped collapsing and expanding of the paddle structure by a movement of the first paddle wing and the second paddle wing. The ligament may extend along the whole length of the paddle structure or only along a section thereof. Generally, the stability of the paddle structure is increased with increasing length along which the ligament extends. The ligament may be arranged centrally within the paddle structure to stabilize the first paddle wing and the second paddle wing in the same manner. Since the resilient element is connected to the ligament on the one side and the first paddle wing and/or the second paddle wing on the other side, a force transmitted by the resilient element onto the first paddle wing and/or the second paddle wing is promoted by the ligament, acting as support for the resilient member and/or the first paddle wing and/or the second paddle wing.

Since the first paddle wing and/or the second paddle wing are indirectly connected to the ligament via the at least one resilient element, it is not necessary that the first paddle wing and/or the second paddle wing are also directly connected to the ligament. In an embodiment, however, the first paddle wing and/or the second paddle wing are directly mechanically coupled with the ligament. Then, there is a direct connection between the first paddle wing and the second paddle wing on the one hand and the ligament on the other hand, as well as an indirect connection via the at least one resilient element. The kind of connection between the first paddle wing and the second paddle wing on the one hand and the ligament on the other hand influences the folding/unfolding mechanism to transfer the paddle structure from its expanded state to its collapsed state, and vice versa. The type of connection also influences the forces acting between the first paddle wing and the second paddle wing on the one hand and the ligament on the other hand. Consequently, depending on the type of connection, the elastic properties of the at least one resilient element can be adjusted to the respective needs in order to achieve an easy collapsing of the paddle structure as well as a reliable expansion of the paddle structure.

In an embodiment, the paddle structure comprises two (or more) resilient elements. In this context, the first of the two (or more) resilient elements is connected to the ligament and the first paddle wing. Likewise, the second of the two (or more) resilient elements is connected to the ligament and the second paddle wing. In this embodiment, not only a single resilient element is responsible for transferring the first paddle wing and the second paddle wing to their expanded states. Rather, two (or more) resilient elements take over this functionality.

In an embodiment, the first of the two (or more) resilient elements and the second of the two (or more) resilient elements are identical in construction. This further facilitates the overall manufacturing of the paddle lead and guarantees for equally distributed forces applied to the first paddle wing and the second paddle wing by the first resilient element and the second resilient element.

In an embodiment, the paddle structure comprises at least two resilient elements, wherein each of the at least two resilient elements extends only along a longitudinal section of the paddle structure. Furthermore, the at least two resilient elements are arranged longitudinally one behind the other. According to this embodiment, a stabilization of the paddle structure and a biasing of the first paddle wing and/or the second paddle wing to its expanded state is achieved with only section-wise arranged resilient elements. In this context, a total number of two, three or four, in particular three, resilient elements is particularly appropriate. Small gaps between the individual resilient elements typically do not have any negative impact on the expansion/collapsing properties of the paddle structure, but further reduce the required material for the elastic element. Typically, the individual resilient elements are equally spaced apart from each other and are equally distributed over the whole length of the paddle structure to achieve a good stabilization of the paddle structure as well as a proper expansion of the first paddle wing and the second paddle wing.

While this section-wise arrangement of resilient elements is typically fully sufficient for most applications, a particularly high shape stability can be achieved if the resilient element extends along the whole length of the paddle structure. In such a case, the resilient element is typically formed as a single part and not divided into individual sections that are longitudinally arranged one behind the other. Such an arrangement of the resilient element along the whole length of the paddle structure is also encompassed by an embodiment of the present invention.

In an aspect, the present invention relates to a method for manufacturing a paddle lead according to the preceding explanations. This method comprises the steps explained in the following.

A lead body is provided, wherein the lead body comprises a plurality of conductors. These conductors extend from a proximal portion of the lead body to a distal portion of the lead body. Additionally, a paddle structure is arranged distally of the lead body and is electrically coupled to the distal portion of the lead body. The paddle structure has a longitudinal extension direction. The paddle structure further comprises a first paddle wing and a second paddle wing. The first paddle wing and the second paddle wing are movable with respect to each other and can be present in an expanded state (implantation state) or in a collapsed state (delivery state).

A plurality of first electrode poles is arranged on the first paddle wing. In this context, each of the first electrode poles is electrically coupled to at least one (and typically exactly one) of the plurality of conductors. Likewise, a plurality of second electrode poles is arranged on the second paddle wing. Also in this case, each of the second electrode poles is electrically coupled to at least one (and typically exactly one) of the plurality of conductors.

The paddle structure further comprises at least one resilient element that is connected to the first paddle wing and/or the second paddle wing. In this context, the at least one resilient member biases the first paddle wing and/or the second paddle wing to the expanded state.

The at least one resilient element has a longitudinal extension direction running exclusively along the longitudinal extension direction of the paddle structure. In addition, the at least one resilient element has a wall that surrounds either alone or together with another part of the paddle structure a hollow space. In this context, the hollow space also extends exclusively along the longitudinal extension direction of the paddle structure.

In an embodiment, the hollow space is accessible only by a first end of the at least one resilient element and/or a second end of the at least one resilient element, wherein the second end lies opposite the first end along the longitudinal extension direction of the at least one resilient element.

All aspects and embodiments of the paddle electrode can be combined in any desired way and can be transferred either individually or in any arbitrary combination to the manufacturing method. Likewise, all aspects and embodiments of the manufacturing method can be combined in any desired way and can be transferred either individually or in any arbitrary combination to the paddle electrode.

Further details of aspects of the present invention will be explained in the following making reference to exemplary embodiments and accompanying Figures. In the Figures:
- Fig. 1A: shows a schematic depiction of the spinal column of a patient with an implanted spinal cord stimulation device and electrode;
- Fig. 1B: shows a schematic cross-section through the spinal column of the patient shown in Fig. 1A in the region of the spinal cord stimulation electrode;
- Figs. 2A to 2D: show a first embodiment of a paddle electrode in different views and different operational states;
- Figs. 3A to 3E: show a second embodiment of a paddle electrode in different views and different operational states;
- Figs. 4A to 4D: show a third embodiment of a paddle electrode in different views and different operational states;
- Figs. 5A and 5B: show a cross-sectional view of a fourth embodiment of a paddle electrode in different operational states;
- Figs. 5C and 5D: show a cross-sectional view of a fifth embodiment of a paddle electrode in different operational states;
- Figs. 5E and 5F: show a cross-sectional view of a sixth embodiment of a paddle electrode in different operational states;
- Figs. 5G and 5H: show a cross-sectional view of a seventh embodiment of a paddle electrode in different operational states;
- Figs. 6A to 6F: show different steps of an implantation procedure for implanting a paddle electrode through an insertion tool.

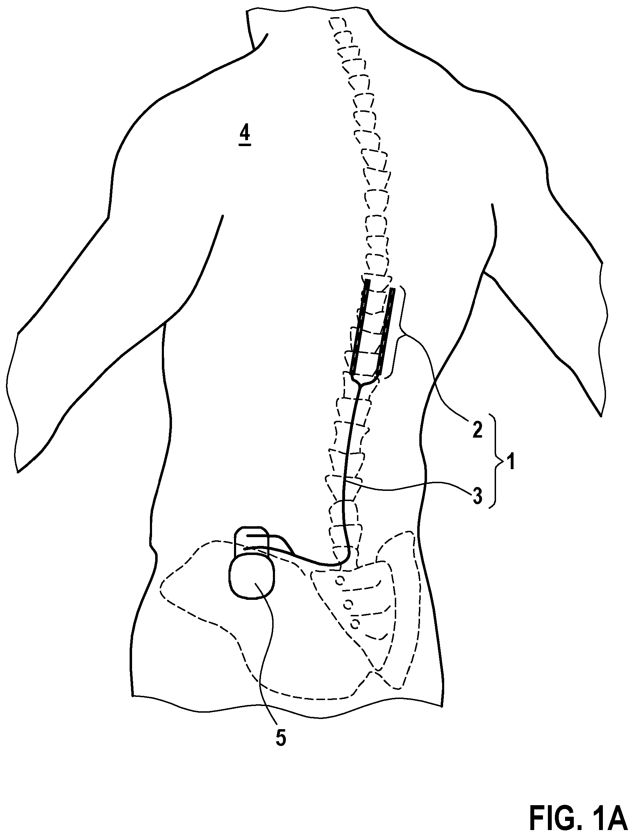

Fig. 1A shows an embodiment of a collapsible paddle lead 1 in its implanted state. In this implanted state, a paddle structure 2 located distally of a lead body 3 is implanted within the epidural space of a patient 4. For this purpose, the paddle lead 1 is, in its collapsed state, introduced into the epidural space in the area of the lumbar spine and then advanced through the epidural tissue to the intended site of implantation. There, it is transferred to its expanded state shown in Fig. 1A. The lead body 3 is connected to a spinal cord stimulation device 5 that is also implanted to the patient 4. The outer diameter of the paddle lead 1 in its collapsed state lies typically in a range between 1.3 mm and 5 mm. In the embodiment of Fig. 1A, the diameter is below 2 mm.

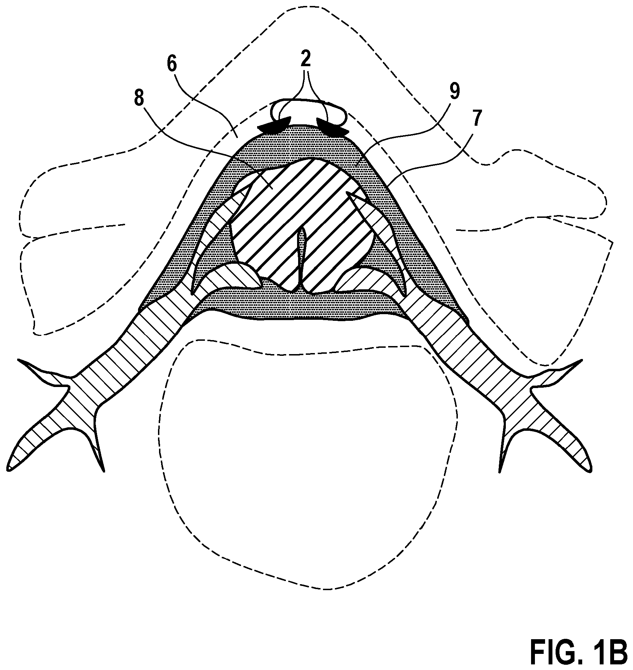

Fig. 1B shows a cross-sectional view through the spinal cord of the patient 4. In this and in all following figures, similar elements will be denoted with the same numeral reference. Here, it can well be seen that the paddle structure 2 is implanted within the epidural space 6 and abuts the dura mater 7 for a stimulation of the spinal cord 8. For this purpose, electric pulses delivered by the electrode poles of the paddle structure 2 are transmitted through the liquor 9 into the spinal cord 8.

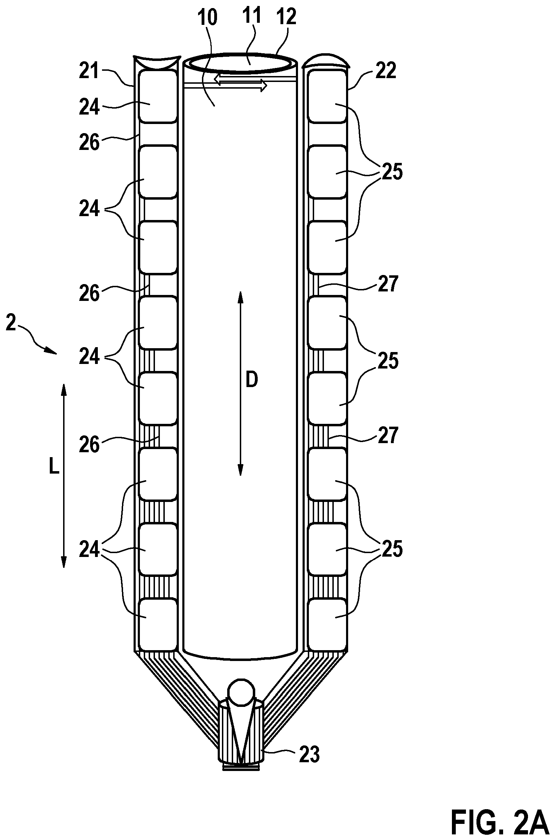

Fig. 2A shows a first embodiment of a paddle structure 2 of a paddle lead 1. The paddle structure 2 extends along a longitudinal extension direction L and comprises a first paddle wing 21 and a second paddle wing 22. At the proximal end of the first paddle wing 21 and the second paddle wing 22, both paddle wings 21, 22 are connected with each other by a switch 23. The switch 23 connects the paddle structure 2 with an lead body 3 (cf. Fig. 1A).

Eight first electrode poles 24 are disposed on a surface of the first paddle wing 21. Likewise, eight second electrode poles 25 are disposed on a surface of the second paddle wing 22.

Each of the first electrode poles 24 is electrically coupled to a first conductor 26. For illustration purposes only, not all of the first conductors 26 are marked with the respective numeral reference.

Likewise, each of the second electrode poles 25 is electrically coupled to a second conductor 27. Also in this case, only some of the second conductors 27 are marked with the respective numeral reference.

The first conductors 26 and the second conductors 27 are guided towards the switch 23 and are then further guided through the lead body 3 to a proximal terminus of the paddle lead 1.

A hollow silicone hose 10 serving as resilient element is placed between and connected to the first paddle wing 21 and the second paddle wing 22. This silicone hose 10 extends in a longitudinal extension direction D that runs exclusively along the longitudinal extension direction L of the paddle structure 2. The silicone hose 10 biases the first paddle wing 21 and the second paddle wing 22 to their expanded states, as shown in Fig. 2A. If the first paddle wing 21 and the second paddle wing 22 are to be transferred into their collapsed state, a movement along a direction indicated by the arrows in Fig. 2A is necessary. At the same time, the silicone hose 10 needs to be compressed. Due to its hollow space 11, which is fully surrounded by a wall 12 of the silicone hose 10, such compression is easily possible.

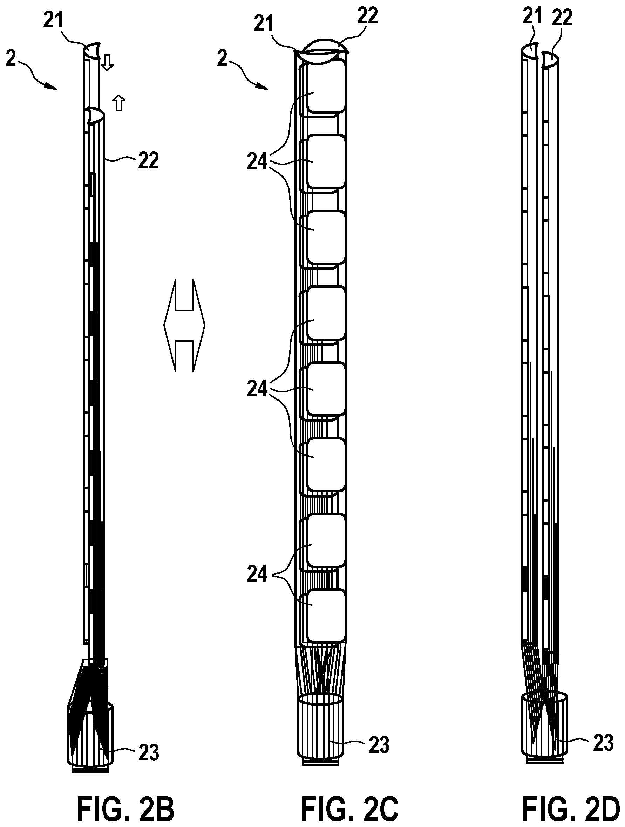

Fig. 2B shows the paddle structure 2 of Fig. 2A in a side view. For illustration purposes only, the silicone hose 10 is not depicted in Fig. 2B. It can be seen from Fig. 2B that a movement of the first electrode wing 21 and the second electrode wing 22 in a scissor-like manner towards each other needs to be performed in order to transfer the paddle structure 2 into its collapsed state. This collapsed state is illustrated in Fig. 2C in a front view and in Fig. 2D in a side view. Also in these illustrations, the silicone hose 10 is omitted for a better visualization.

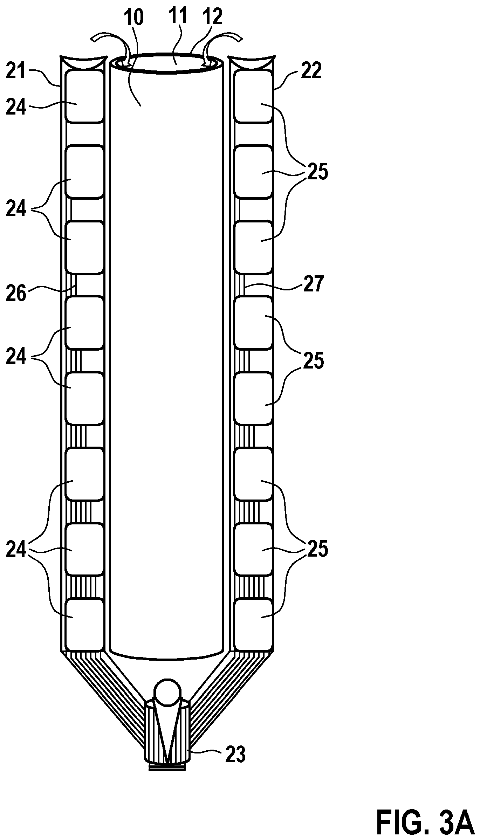

Figs. 3A to 3E show a second embodiment of a paddle structure 2 that is very similar to the embodiment shown in Figs. 2A to 2D. Therefore, reference is made to the above explanations. The main difference between the embodiment shown in Figs. 3A to 3E and the embodiment shown in Figs. 2A to 2D is the folding/unfolding mechanism of the paddle structure.

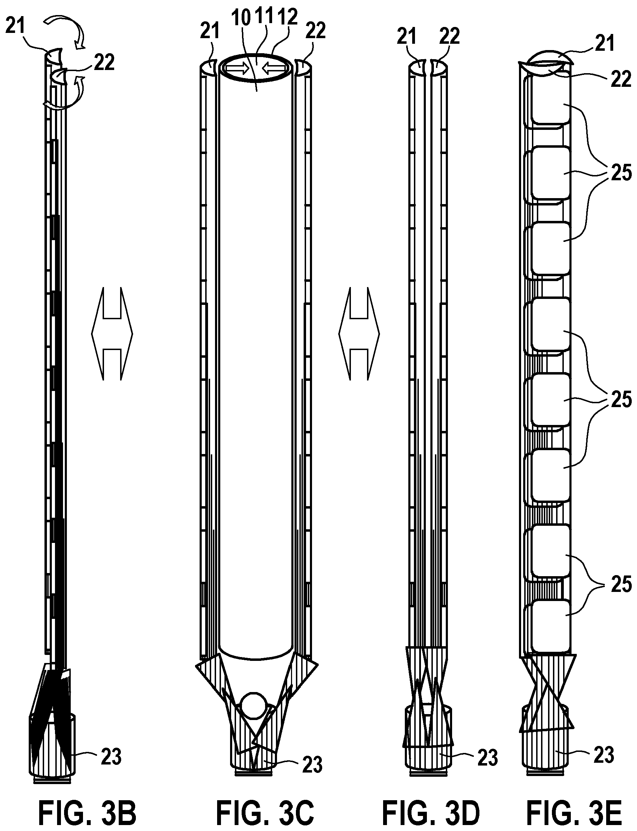

In the embodiment shown in Figs. 3A to 3E, the first paddle wing 21 and the second paddle wing 22 are first tilted by 90° when they are transferred into their collapsed state. An arrangement as shown in Fig. 3C results. Then, the first paddle wing 21 and the second paddle wing 22 are pressed against each other in a V-like manner to be transferred into their collapsed state shown in Figs. 3D and 3E. For simplification purposes, the silicone hose 10 is not visualized in Figs. 3B, 3D and 3E.

As in case of the embodiment shown in Figs. 2A to 2D, the silicone hose 10 serves for a pretensioning of the first paddle wing 21 and the second paddle wing 22 into their expanded state (as shown in Figs. 3A and 3B), as long as no higher compressive force (acting in the direction indicated by the arrows in Fig. 3C) acts upon the silicone hose 10.

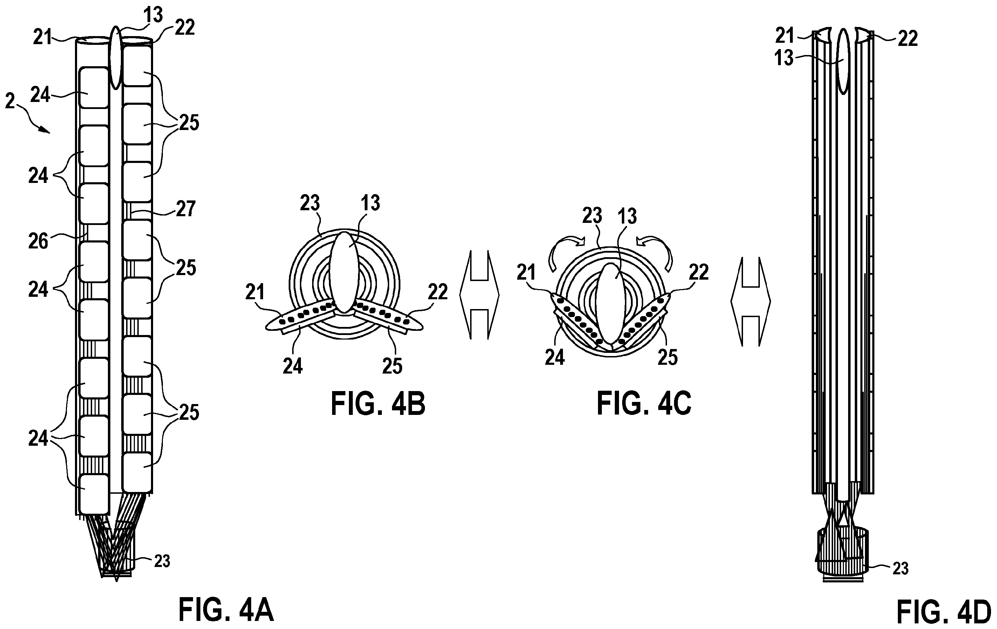

Figs. 4A to 4D show a third embodiment of a paddle structure 2. Here, a ligament 13 is arranged between the first paddle wing 21 and the second paddle wing 22. In this context, the first paddle wing 21 and the second paddle wing 22 are mechanically linked to the ligament 13. Consequently, a Y-like shape of the paddle structure 2 results in a cross-sectional view, as shown in Fig. 4B. Upon moving the first paddle wing 21 and the second paddle wing 22 towards the ligament 13 (as shown in Fig. 4C), the collapsed state of the paddle structure 2 results in which the ligament 13 is closely surrounded by the first paddle wing 21 and the second paddle wing 22.

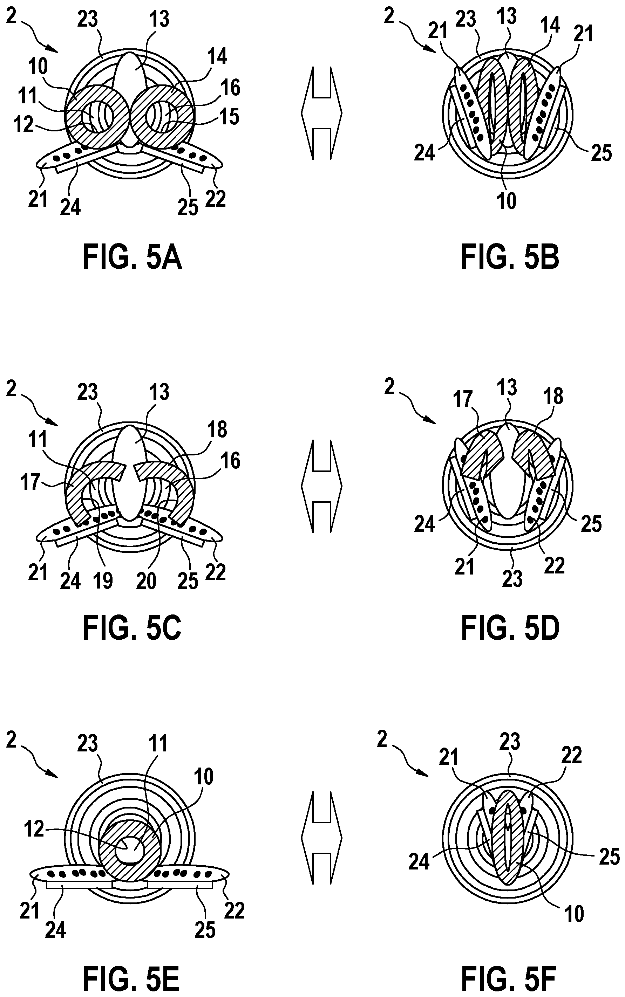

The embodiment shown in Figs. 4A to 4D also comprises a resilient element biasing the first paddle wing 21 and the second paddle wing 22 to the respective expanded states. For simplification purposes only, this is not illustrated in Figs. 4A to 4D, but will be explained in detail with respect to Figs. 5A to 5D.

Figs. 5A and 5B show a fourth embodiment of a paddle structure 2 that is folded in a Y-like manner, as explained with respect to Figs. 4A to 4D. Here, the first paddle wing 21 is connected via a first silicone hose 10 to a ligament 13. Likewise, the second paddle wing 22 is connected via a second silicone hose 14 to the ligament 13. At the same time, the first paddle wing 21 and the second paddle wing 22 are not directly connected with the ligament 13. By folding the first paddle wing 21 and the second paddle wing 22 towards the ligament 13 under compression of the first silicone hose 10 and the second silicon hose 14, the paddle structure 2 is transferred into its compressed (collapsed) state in which it has a practically isodiametric outer circumference as can well be seen in Fig. 5B by comparing the space needed by the paddle structure 2 and the space needed by the switch 23.

The first silicon hose 10 surrounds with its inner wall 12 a first hollow space 11. Likewise, the second silicon hose 14 surrounds with an inner wall 15 a second hollow space 16. The first hollow space 11 and the second hollow space 16 allow a very easy compression of the first silicon hose 10 and the second silicon hose 14.

Figs. 5C and 5D show an embodiment that is very similar to the embodiment of Figs. 5A and 5B. The main difference is that a first silicon hose section 17 and a second silicon hose section 18 are used as resilient elements instead of the first silicon hose 10 and the second silicon hose 14. The first silicon hose section 17 surrounds with its inner wall 19 together with the first paddle wing 21 and the ligament a first hollow space 11. Likewise, the second silicon hose section 18 surrounds with its inner wall 20 together with the second paddle wing 22 and the ligament 13 a second hollow space 16. By folding the first paddle wing 21 and the second paddle wing 22 towards the ligament 13 under compression of the first silicon hose section 17 and the second silicon hose section 18, the paddle structure 2 is transferred into its collapsed state. Here, the first silicon hose section 17 and the second silicon hose section 18 do no longer form a hollow space with the ligament 13 and the first paddle wing 21 or the second paddle wing 22, respectively. Rather, due to the relative movement of the first paddle wing 21 and the second paddle wing 22 with respect to the ligament 13, the first hollow space 11 and the second hollow space 16 have been disintegrated. However, a presence of the first hollow space 11 and the second hollow space 16 is only necessary in at least one operational state (in particular in the expanded state) of the paddle structure 2 to allow for a good collapsibility of the paddle structure 2.

Figs. 5E and 5F show another embodiment of a paddle structure 2 in a cross-sectional view. In this embodiment, no ligament 13 like in the embodiments shown in Figs. 5A to 5D is present. Rather, a single silicon hose 10 is arranged between the first paddle wing 21 and the second paddle wing 22 and is connected to both the first paddle wing 21 and the second paddle wing 22. By folding the first paddle wing 21 and the second paddle wing 22 towards each other under compression of the silicon hose 10, the paddle structure 2 is transferred into its collapsed state shown in Fig. 5F. The space requirement of the paddle structure 2 in this collapsed state is even smaller than in case of the embodiments shown in Figs. 5A to 5D since there is no space requirement for a ligament.

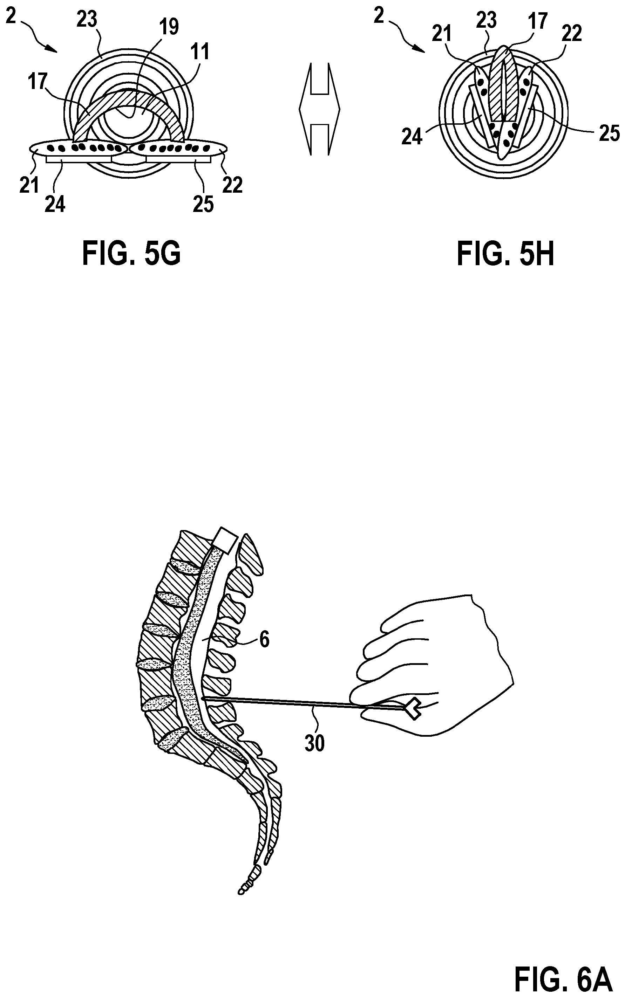

Figs. 5G and 5H show another embodiment of a paddle structure 2 in which the resilient element is not formed by a silicon hose, but rather by a silicon hose section 17 (like in case of the embodiment shown in Figs. 5C and 5D). However, also the embodiment shown in Figs. 5G and 5H does not comprise a ligament. Rather, the first paddle wing 21 and the second paddle wing 22 are directly connected with each other via the silicon hose section 17. A hollow space 11 is surrounded by an inner wall 19 of the silicon hose section 17 as well as the walls of the first paddle wing 21 and the second paddle wing 22. This paddle structure 2 can be transferred to its collapsed state like the paddle structure 2 shown in Figs. 5E and 5F. The collapsed state is illustrated in Fig. 5H. An outer diameter of the paddle structure 2 is smaller than the outer diameter of the switch 23 located proximally of the paddle structure 2. Consequently, a paddle lead comprising such a paddle structure 2 is also well suited to be delivered to an implantation site with the help of an insertion tool.

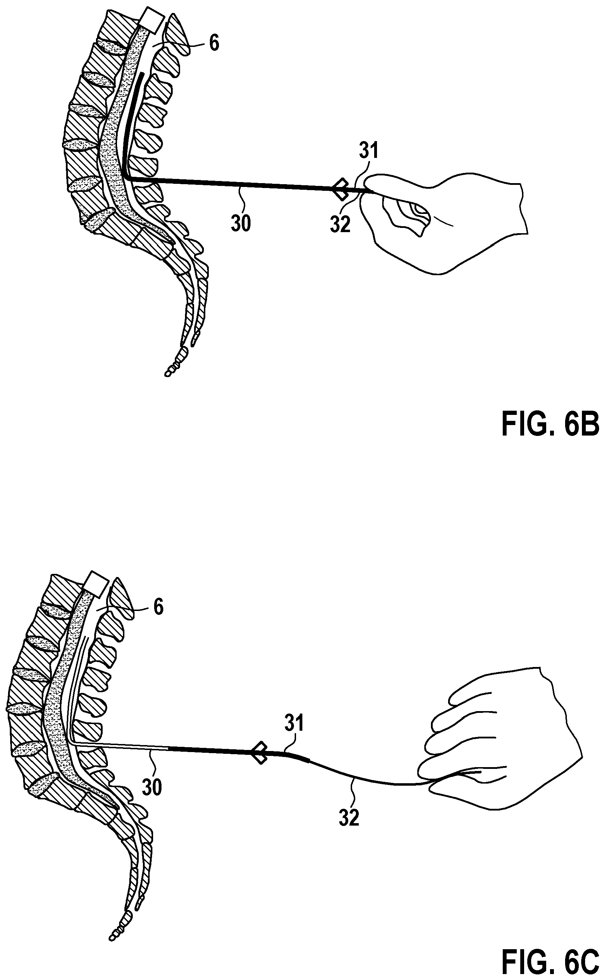

Figs. 6A to 6F illustrate an according implantation procedure. In a first step, an epidural cannula 30 is inserted into the epidural space 6 of the patient 4 (cf. Fig. 6A). Afterwards, a guiding catheter 31 is inserted into the epidural cannula 30, wherein a guide wire 32 is located inside the guiding catheter 31. The guiding catheter 31 is advanced to the intended site of implantation (cf. Fig. 6B).

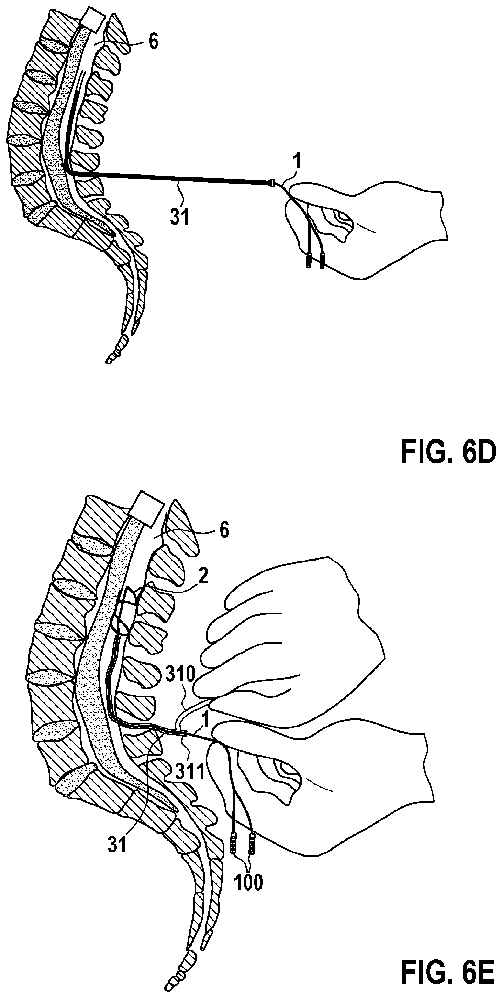

Afterwards, the guide wire 32 is removed from the guiding catheter 31 (cf. Fig. 6C). Then, a paddle lead 1 is inserted in its collapsed state into the guiding catheter 31 (cf. Fig. 6D). At the intended site of implantation, a paddle structure 2 of the paddle lead 1 exits the guiding catheter 31 and is transferred by a resilient element (like the silicon hose 10 referred to in previous exemplary embodiments) into its expanded state. The paddle structure 2 is then fixed at its implantation site. The guiding catheter 31 is then retracted in a proximal direction. To separate the guiding catheter 31 from the paddle lead 1, the guiding catheter 31 is slit with a slitting tool into a first guiding catheter part 310 and a second guiding catheter part 311 (cf. Fig. 6E).

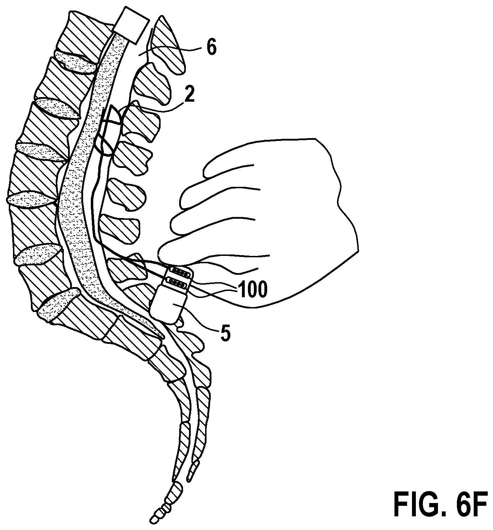

In a final step, plugs 100 of the paddle lead 1 are connected to a spinal cord stimulation device 5 acting as active implant (cf. Fig. 6F). At this stage, the expandable paddle lead 1 is ready to be used for spinal cord stimulation via the implanted paddle structure 2.

## Claims

1. Paddle lead (1) for implantation in the epidural space through an insertion tool, the paddle lead (1) comprising:
a lead body (3) comprising a plurality of conductors (26, 27) extending from a proximal portion of the lead body (3) to a distal portion of the lead body (3);
a paddle structure (2) electrically coupled to the distal portion of the lead body (3) and arranged distally of the lead body (3), wherein the paddle structure (2) has a longitudinal extension direction (L) and comprises:
a first paddle wing (21) and a second paddle wing (22), wherein the first paddle wing (21) and the second paddle wing (22) are movable with respect to each other and can be present in an expanded state and in a collapsed state;
a plurality of first electrode poles (24) arranged on the first paddle wing (21), wherein each of the first electrode poles (24) is electrically coupled to at least one of the plurality of conductors (26, 27);
a plurality of second electrode poles (25) arranged on the second paddle wing (22), wherein each of the second electrode poles (25) is electrically coupled to at least one of the plurality of conductors (26, 27);
at least one resilient element (10, 14, 17, 18) connected with at least one of the first paddle wing (21) and the second paddle wing (22), wherein the at least one resilient member (10, 14, 17, 18) biases at least one of the first paddle wing (21) and the second paddle wing (22) to the expanded state;
wherein the at least one resilient element (10, 14, 17, 18) has a longitudinal extension direction (D) that runs exclusively along the longitudinal extension direction (L) of the paddle structure (2) and in that the at least one resilient element (10, 14, 17, 18) has a wall (12, 15, 19, 20) that surrounds alone or with another part of the paddle structure (2) a hollow space (11, 16), wherein the hollow space (11, 16) also extends exclusively along the longitudinal extension direction (L) of the paddle structure (2).

2. Paddle lead according to claim 1, **characterized in that** the at least one resilient element (10, 14, 17, 18) is made from a different material than the first paddle wing (21) and the second paddle wing (22).

3. Paddle lead according to claim 1 or 2, **characterized in that** the at least one resilient element (10, 14, 17, 18) is made from an elastic plastic material.

4. Paddle lead according to any of the preceding claims, **characterized in that** the at least one resilient element (10, 14, 17, 18) has a circular or elliptic cross section.

5. Paddle lead according to any of claims 1 to 3, **characterized in that** the at least one resilient element (10, 14, 17, 18) has a cross section of a segment of a circle or of a segment of an ellipse.

6. Paddle lead according to any of the preceding claims, **characterized in that** the first paddle wing (21) and the second paddle wing (22) are connected with each other in a proximal portion of the first paddle wing (21) and the second paddle wing (22), but unconnected in a distal portion of the first paddle wing (21) and the second paddle wing (22).

7. Paddle lead according to any of the preceding claims, **characterized in that** the at least one resilient element (10, 14, 17, 18) is connected with the first paddle wing (21) and the second paddle wing (22).

8. Paddle lead according to any of claims 1 to 6, **characterized in that** the paddle structure (2) additionally comprises a ligament (13) extending along the longitudinal extension direction (L) of the paddle structure (2), wherein the ligament (13) is arranged between the first paddle wing (21) and the second paddle wing (22), wherein the at least one resilient element (10, 14, 17, 18) is also connected to the ligament (13).

9. Paddle lead according to claim 8, **characterized in that** the ligament (13) is connected to the first paddle wing (21) and the second paddle wing (22).

10. Paddle lead according to claim 8 or 9, **characterized in that** the paddle structure (2) comprises two resilient elements (10, 14; 17, 18), wherein a first (10, 17) of the two resilient elements (10, 14; 17, 18) is connected to the ligament (13) and the first paddle wing (21), and wherein a second (14, 18) of the two resilient elements (10, 14; 17, 18) is connected to the ligament (13) and the second paddle wing (22).

11. Paddle lead according to claim 10, **characterized in that** the first (10, 17) of the two resilient elements (10, 14; 17, 18) and the second (14, 18) of the two resilient elements (10, 14; 17, 18) are identical in construction.

12. Paddle lead according to any of the preceding claims, **characterized in that** the paddle structure (2) comprises at least two resilient elements (10, 14, 17, 18), wherein each of the at least two resilient elements (10, 14, 17, 18) extends only along a longitudinal section of the paddle structure (2), and wherein the at least two resilient elements (10, 14, 17, 18) are arranged longitudinally one behind the other.

13. Paddle lead according to any of claims 1 to 11, **characterized in that** the at least one resilient element (10, 14, 17, 18) extends along a whole length of the paddle structure (2).

14. Paddle lead according to any of the preceding claims, **characterized in that** the hollow space (11, 16) is accessible only via a first end of the at least one resilient element (10, 14, 17, 18) and/or a second end of the at least one resilient element (10, 14, 17, 18), the second end lying opposite the first end along the longitudinal extension direction (D) of the at least one resilient element (10, 14, 17, 18)

15. Method for manufacturing a paddle lead (1) according to any of the preceding claims, the method comprising the following steps:
providing an lead body (3) comprising a plurality of conductors (26, 27) extending from a proximal portion of the lead body (3) to a distal portion of the lead body (3);
arranging a paddle structure (2) distally of the lead body (3) and electrically coupling it to the distal portion of the lead body (3), wherein the paddle structure (2) has a longitudinal extension direction (L) and comprises:
a first paddle wing (21) and a second paddle wing (22), wherein the first paddle wing (21) and the second paddle wing (22) are movable with respect to each other and can be present in an expanded state and in a collapsed state;
a plurality of first electrode poles (24) arranged on the first paddle wing (21), wherein each of the first electrode poles (24) is electrically coupled to at least one of the plurality of conductors (26, 27);
a plurality of second electrode poles (25) arranged on the second paddle wing (22), wherein each of the second electrode poles (25) is electrically coupled to at least one of the plurality of conductors (26, 27);
at least one resilient element (10, 14, 17, 18) connected with at least one of the first paddle wing (21) and the second paddle wing (22), wherein the at least one resilient member (10, 14, 17, 18) biases at least one of the first paddle wing (21) and the second paddle wing (22) to the expanded state;
wherein the at least one resilient element (10, 14, 17, 18) has a longitudinal extension direction (D) that runs exclusively along the longitudinal extension direction (L) of the paddle structure (2) and in that the at least one resilient element (10, 14, 17, 18) has a wall (12, 15, 19, 20) that surrounds alone or with another part of the paddle structure a hollow space (11, 16), wherein the hollow space (11, 16) also extends exclusively along the longitudinal extension direction (L) of the paddle structure (2).

## Patentansprüche

1. Paddelelektrode (1) zur Implantation in den Epiduralraum durch ein Einführinstrument, wobei die Paddelelektrode (1) umfasst:
einen Leitungskörper (3), der eine Vielzahl von Leitern (26, 27) umfasst, die sich von einem proximalen Abschnitt des Leitungskörpers (3) zu einem distalen Abschnitt des Leitungskörpers (3) erstrecken;
eine Paddelstruktur (2), die elektrisch mit dem distalen Abschnitt des Leitungskörpers (3) gekoppelt und distal vom Leitungskörper (3) angeordnet ist, wobei die Paddelstruktur (2) eine Längserstreckungsrichtung (L) aufweist und umfasst:
einen ersten Paddelflügel (21) und einen zweiten Paddelflügel (22), wobei der erste Paddelflügel (21) und der zweite Paddelflügel (22) in Bezug zueinander beweglich sind und in einem expandierten Zustand und in einem zusammengeklappten Zustand vorliegen können;
eine Vielzahl von ersten Elektrodenpolen (24), die auf dem ersten Paddelflügel (21) angeordnet sind, wobei jeder der ersten Elektrodenpole (24) elektrisch mit wenigstens einem der Vielzahl von Leitern (26, 27) verbunden ist;
eine Vielzahl von zweiten Elektrodenpolen (25), die auf dem zweiten Paddelflügel (22) angeordnet sind, wobei jeder der zweiten Elektrodenpole (25) elektrisch mit wenigstens einem der Vielzahl von Leitern (26, 27) verbunden ist;
wenigstens ein federndes Element (10, 14, 17, 18), das mit wenigstens einem von dem ersten Paddelflügel (21) und dem zweiten Paddelflügel (22) verbunden ist, wobei das wenigstens eine federnde Element (10, 14, 17, 18) wenigstens einen von dem ersten Paddelflügel (21) und dem zweiten Paddelflügel (22) in den expandierten Zustand vorspannt;
wobei das wenigstens eine federnde Element (10, 14, 17, 18) eine Längserstreckungsrichtung (D) aufweist, die ausschließlich entlang der Längserstreckungsrichtung (L) der Paddelstruktur (2) verläuft, und wobei das wenigstens eine federnde Element (10, 14, 17, 18) eine Wand (12, 15, 19, 20) aufweist, die allein oder mit einem anderen Teil der Paddelstruktur (2) einen Hohlraum (11, 16) umschließt, wobei sich der Hohlraum (11, 16) ebenfalls ausschließlich entlang der Längserstreckungsrichtung (L) der Paddelstruktur (2) erstreckt.

2. Paddelelektrode nach Anspruch 1, **dadurch gekennzeichnet, dass** das wenigstens eine federnde Element (10, 14, 17, 18) aus einem anderen Material als der erste Paddelflügel (21) und der zweite Paddelflügel (22) hergestellt ist.

3. Paddelelektrode nach Anspruch 1 oder 2, **dadurch gekennzeichnet, dass** das wenigstens eine federnde Element (10, 14, 17, 18) aus einem elastischen Kunststoffmaterial hergestellt ist.

4. Paddelelektrode nach einem der vorstehenden Ansprüche, **dadurch gekennzeichnet, dass** das wenigstens eine federnde Element (10, 14, 17, 18) einen kreisförmigen oder elliptischen Querschnitt aufweist.

5. Paddelelektrode nach einem der Ansprüche 1 bis 3, **dadurch gekennzeichnet, dass** das wenigstens eine federnde Element (10, 14, 17, 18) einen Querschnitt eines Kreissegments oder eines Ellipsensegments aufweist.

6. Paddelelektrode nach einem der vorstehenden Ansprüche, **dadurch gekennzeichnet, dass** der erste Paddelflügel (21) und der zweite Paddelflügel (22) in einem proximalen Abschnitt des ersten Paddelflügels (21) und des zweiten Paddelflügels (22) miteinander verbunden sind, jedoch in einem distalen Abschnitt des ersten Paddelflügels (21) und des zweiten Paddelflügels (22) nicht verbunden sind.

7. Paddelelektrode nach einem der vorstehenden Ansprüche, **dadurch gekennzeichnet, dass** das wenigstens eine federnde Element (10, 14, 17, 18) mit dem ersten Paddelflügel (21) und dem zweiten Paddelflügel (22) verbunden ist.

8. Paddelelektrode nach einem der Ansprüche 1 bis 6, **dadurch gekennzeichnet, dass** die Paddelstruktur (2) zusätzlich ein Band (13) umfasst, das sich entlang der Längserstreckungsrichtung (L) der Paddelstruktur (2) erstreckt, wobei das Band (13) zwischen dem ersten Paddelflügel (21) und dem zweiten Paddelflügel (22) angeordnet ist, wobei das wenigstens eine federnde Element (10, 14, 17, 18) ebenfalls mit dem Band (13) verbunden ist.

9. Paddelelektrode gemäß Anspruch 8, **dadurch gekennzeichnet, dass** das Band (13) mit dem ersten Paddelflügel (21) und dem zweiten Paddelflügel (22) verbunden ist.

10. Paddelelektrode nach Anspruch 8 oder 9, **dadurch gekennzeichnet, dass** die Paddelstruktur (2) zwei federnde Elemente (10, 14; 17, 18) umfasst, wobei ein erstes (10, 17) der beiden federnden Elemente (10, 14; 17, 18) mit dem Band (13) und dem ersten Paddelflügel (21) verbunden ist und wobei ein zweites (14, 18) der beiden federnden Elemente (10, 14; 17, 18) mit dem Band (13) und dem zweiten Paddelflügel (22) verbunden ist.

11. Paddelelektrode gemäß Anspruch 10, **dadurch gekennzeichnet, dass** das erste (10, 17) der beiden federnden Elemente (10, 14; 17, 18) und das zweite (14, 18) der beiden federnden Elemente (10, 14; 17, 18) baugleich sind.

12. Paddelelektrode nach einem der vorstehenden Ansprüche, **dadurch gekennzeichnet, dass** die Paddelstruktur (2) wenigstens zwei federnde Elemente (10, 14, 17, 18) umfasst, wobei sich jedes der wenigstens zwei federnden Elemente (10, 14, 17, 18) nur entlang eines Längsabschnitts der Paddelstruktur (2) erstreckt und wobei die wenigstens zwei federnden Elemente (10, 14, 17, 18) in Längsrichtung hintereinander angeordnet sind.

13. Paddelelektrode nach einem der Ansprüche 1 bis 11, **dadurch gekennzeichnet, dass** sich das wenigstens eine federnde Element (10, 14, 17, 18) entlang einer gesamten Länge der Paddelstruktur (2) erstreckt.

14. Paddelelektrode nach einem der vorstehenden Ansprüche, **dadurch gekennzeichnet, dass** der Hohlraum (11, 16) nur über ein erstes Ende des wenigstens einen federnden Elements (10, 14, 17, 18) und/oder ein zweites Ende des wenigstens einen federnden Elements (10, 14, 17, 18) zugänglich ist, wobei das zweite Ende entlang der Längserstreckungsrichtung (D) des wenigstens einen federnden Elements (10, 14, 17, 18) dem ersten Ende gegenüberliegt.

15. Verfahren zur Herstellung einer Paddelelektrode (1) nach einem der vorstehenden Ansprüche, wobei das Verfahren die folgenden Schritte umfasst:
Bereitstellen eines Leitungskörpers (3), der eine Vielzahl von Leitern (26, 27) umfasst, die sich von einem proximalen Abschnitt des Leitungskörpers (3) zu einem distalen Abschnitt des Leitungskörpers (3) erstrecken;
Anordnen einer Paddelstruktur (2) distal zum Leitungskörper (3) und elektrisches Koppeln derselben mit dem distalen Abschnitt des Leitungskörpers (3), wobei die Paddelstruktur (2) eine Längserstreckungsrichtung (L) aufweist und umfasst:
einen ersten Paddelflügel (21) und einen zweiten Paddelflügel (22), wobei der erste Paddelflügel (21) und der zweite Paddelflügel (22) in Bezug zueinander beweglich sind und in einem expandierten Zustand und in einem zusammengeklappten Zustand vorliegen können;
eine Vielzahl von ersten Elektrodenpolen (24), die auf dem ersten Paddelflügel (21) angeordnet sind, wobei jeder der ersten Elektrodenpole (24) elektrisch mit wenigstens einem der Vielzahl von Leitern (26, 27) verbunden ist;
eine Vielzahl von zweiten Elektrodenpolen (25), die auf dem zweiten Paddelflügel (22) angeordnet sind, wobei jeder der zweiten Elektrodenpole (25) elektrisch mit wenigstens einem der Vielzahl von Leitern (26, 27) verbunden ist;
wenigstens ein federndes Element (10, 14, 17, 18), das mit wenigstens einem von dem ersten Paddelflügel (21) und dem zweiten Paddelflügel (22) verbunden ist, wobei das wenigstens eine federnde Element (10, 14, 17, 18) wenigstens einen von dem ersten Paddelflügel (21) und dem zweiten Paddelflügel (22) in den expandierten Zustand vorspannt;
wobei das wenigstens eine federnde Element (10, 14, 17, 18) eine Längserstreckungsrichtung (D) aufweist, die ausschließlich entlang der Längserstreckungsrichtung (L) der Paddelstruktur (2) verläuft, und wobei das wenigstens eine federnde Element (10, 14, 17, 18) eine Wand (12, 15, 19, 20) aufweist, die allein oder mit einem anderen Teil der Paddelstruktur einen Hohlraum (11, 16) umschließt, wobei sich der Hohlraum (11, 16) ebenfalls ausschließlich entlang der Längserstreckungsrichtung (L) der Paddelstruktur (2) erstreckt.

## Revendications

1. Électrode en palette (1) destiné à une implantation dans l'espace péridural au travers d'un outil d'insertion, l'électrode en palette (1) comprenant :
un corps de câble (3) comprenant une pluralité de conducteurs (26, 27) s'étendant à partir d'une partie proximale du corps de câble (3) jusqu'à une partie distale du corps de câble (3) ;
une structure de palette (2) couplée électriquement à la partie distale du corps de câble (3) et agencée de manière distale par rapport au corps de câble (3), dans lequel la structure de palette (2) a une direction d'extension longitudinale (L) et comprend :
une première ailette de palette (21) et une seconde ailette de palette (22), dans lequel la première ailette de palette (21) et la seconde ailette de palette (22) peuvent être déplacées l'une par rapport à l'autre et peuvent être présentes dans un état étendu et dans un état rétracté ;
une pluralité de premiers pôles d'électrode (24) agencés sur la première ailette de palette (21), dans lequel chacun des premiers pôles d'électrode (24) est couplé électriquement à au moins un de la pluralité de conducteurs (26, 27) ;
une pluralité de seconds pôles d'électrode (25) agencés sur la seconde ailette de palette (22), dans lequel chacun des seconds pôles d'électrode (25) est couplé électriquement à au moins un de la pluralité de conducteurs (26, 27) ;
au moins un élément élastique (10, 14, 17, 18) connecté avec au moins une parmi la première ailette de palette (21) et la seconde ailette de palette (22), dans lequel l'au moins un élément élastique (10, 14, 17, 18) dévie au moins une parmi la première ailette de palette (21) et la seconde ailette de palette (22) vers l'état étendu ;
dans lequel
l'au moins un élément élastique (10, 14, 17, 18) a une direction d'extension longitudinale (D) qui court exclusivement le long de la direction d'extension longitudinale (L) de la structure d'ailette (2) et en ce que l'au moins un élément élastique (10, 14, 17, 18) a une paroi (12, 15, 19, 20) qui entoure seul ou avec une autre partie de la structure de palette (2) un espace creux (11, 16), dans lequel l'espace creux (11, 16) s'étend également exclusivement le long de la direction d'extension longitudinale (L) de la structure de palette (2).

2. Électrode en palette selon la revendication 1, **caractérisée en ce que** l'au moins un élément élastique (10, 14, 17, 18) est fabriqué en un matériau différent de la première ailette de palette (21) et de la seconde ailette de palette (22).

3. Électrode en palette selon la revendication 1 ou 2, **caractérisée en ce que** l'au moins un élément élastique (10, 14, 17, 18) est fabriqué à partir d'un matériau plastique élastique.

4. Électrode en palette selon l'une quelconque des revendications précédentes, **caractérisée en ce que** l'au moins un élément élastique (10, 14, 17, 18) a une section transversale circulaire ou elliptique.

5. Électrode en palette selon l'une quelconque des revendications 1 à 3, **caractérisée en ce que** l'au moins un élément élastique (10, 14, 17, 18) a une section transversale d'un segment d'un cercle ou d'un segment d'une ellipse.

6. Électrode en palette selon l'une quelconque des revendications précédentes, **caractérisée en ce que** la première ailette de palette (21) et la seconde ailette de palette (22) sont connectées l'une à l'autre dans une partie proximale de la première ailette de palette (21) et de la seconde ailette de palette (22), mais non connectées dans une partie distale de la première ailette de palette (21) et de la seconde ailette de palette (22).

7. Électrode en palette selon l'une quelconque des revendications précédentes, **caractérisée en ce que** l'au moins un élément élastique (10, 14, 17, 18) est connecté à la première ailette de palette (21) et à la seconde ailette de palette (22).

8. Électrode en palette selon l'une quelconque des revendications 1 à 6, **caractérisée en ce que** la structure de palette (2) comprend de plus un ligament (13) s'étendant le long de la direction d'extension longitudinale (L) de la structure de palette (2), dans lequel le ligament (13) est agencé entre la première ailette de palette (21) et la seconde ailette de palette (22), dans lequel l'au moins un élément élastique (10, 14, 17, 18) est également connecté au ligament (13).

9. Électrode en palette selon la revendication 8, **caractérisée en ce que** le ligament (13) est connecté à la première ailette de palette (21) et à la seconde ailette de palette (22).

10. Électrode en palette selon la revendication 8 ou 9, **caractérisée en ce que** la structure de palette (2) comprend deux éléments élastiques (10, 14 ; 17, 18), dans lequel un premier (10, 17) des deux éléments élastiques (10, 14 ; 17, 18) est connecté au ligament (13) et à la première ailette de palette (21), et dans lequel un second (14, 18) des deux éléments élastiques (10, 14 ; 17, 18) est connecté au ligament (13)et à la seconde ailette de palette (22).

11. Électrode en palette selon la revendication 10, **caractérisée en ce que** le premier (10, 17) des deux éléments élastiques (10, 14 ; 17, 18) et le second (14, 18) des deux éléments élastiques (10, 14 ; 17, 18) sont de construction identique.

12. Électrode en palette selon l'une quelconque des revendications précédentes, **caractérisée en ce que** la structure de palette (2) comprend au moins deux éléments élastiques (10, 14, 17, 18), dans lequel chacun des au moins deux éléments élastiques (10, 14, 17, 18) s'étend uniquement le long d'une section longitudinale de la structure de palette (2), et dans lequel les au moins deux éléments élastiques (10, 14, 17, 18) sont agencés dans la direction longitudinale l'une derrière l'autre.

13. Électrode en palette selon l'une quelconque des revendications 1 à 11, **caractérisée en ce que** l'au moins un élément élastique (10, 14, 17, 18) s'étend le long d'une longueur totale de la structure de palette (2).

14. Électrode en palette selon l'une quelconque des revendications précédentes, **caractérisée en ce que** l'espace creux (11, 16) est accessible uniquement par l'intermédiaire d'une première extrémité de l'au moins un élément élastique (10, 14, 17, 18) et/ou une seconde extrémité de l'au moins un élément élastique (10, 14, 17, 18), la seconde extrémité reposant à l'opposé de la première extrémité le long de la direction d'extension longitudinale (D) de l'au moins un élément élastique (10, 14, 17, 18)

15. Procédé de fabrication d'un électrode en palette (1) selon l'une quelconque des revendications précédentes, le procédé comprenant les étapes suivantes consistant à :
fournir un corps de câble (3) comprenant une pluralité de conducteurs (26, 27) s'étendant à partir d'une partie proximale du corps de câble (3) jusqu'à une partie distale du corps de câble (3) ;
agencer une structure de palette (2) de manière distale par rapport au corps de câble (3) et la coupler électriquement à la partie distale du corps de câble (3), dans lequel la structure de palette (2) a une direction d'extension longitudinale (L) et comprend :
une première ailette de palette (21) et une seconde ailette de palette (22), dans lequel la première ailette de palette (21) et la seconde ailette de palette (22) peuvent être déplacées l'une par rapport à l'autre et peuvent être présentes dans un état étendu et dans un état rétracté ;
une pluralité de premiers pôles d'électrode (24) agencés sur la première ailette de palette (21), dans lequel chacun des premiers pôles d'électrode (24) est couplé électriquement à au moins un de la pluralité de conducteurs (26, 27) ;
une pluralité de seconds pôles d'électrode (25) agencés sur la seconde ailette de palette (22), dans lequel chacun des seconds pôles d'électrode (25) est couplé électriquement à au moins un de la pluralité de conducteurs (26, 27) ;
au moins un élément élastique (10, 14, 17, 18) connecté avec au moins une parmi la première ailette de palette (21) et la seconde ailette de palette (22), dans lequel l'au moins un élément élastique (10, 14, 17, 18) dévie au moins une parmi la première ailette de palette (21) et la seconde ailette de palette (22) vers l'état étendu ;
dans lequel
l'au moins un élément élastique (10, 14, 17, 18) a une direction d'extension longitudinale (D) qui court exclusivement le long de la direction d'extension longitudinale (L) de la structure d'ailette (2) et en ce que l'au moins un élément élastique (10, 14, 17, 18) a une paroi (12, 15, 19, 20) qui entoure seule ou avec une autre partie de la structure de palette un espace creux (11, 16), dans lequel l'espace creux (11, 16) s'étend également exclusivement le long de la direction d'extension longitudinale (L) de la structure de palette (2).
